(19) Europäisches Patentamt
European Patent Office
Office européen des brevets

(11) Veröffentlichungsnummer: **0 140 298**
**B1**

# (12) EUROPÄISCHE PATENTSCHRIFT

(45) Veröffentlichungstag der Patentschrift: **28.01.87**

(51) Int. Cl.⁴: **C 07 C 149/273**, C 08 K 5/37

(21) Anmeldenummer: **84112591.7**

(22) Anmeldetag: **18.10.84**

## (54) Substituierte 4-Hydroxybenzylthioaldehyde und -ketone.

(30) Priorität: **21.10.83 US 544297**

(43) Veröffentlichungstag der Anmeldung: **08.05.85 Patentblatt 85/19**

(45) Bekanntmachung des Hinweises auf die Patenterteilung: **28.01.87 Patentblatt 87/5**

(84) Benannte Vertragsstaaten: **DE FR GB IT**

(56) Entgegenhaltungen:
**EP - A - 0 060 799**
**DE - B - 1 593 821**
**GB - A - 1 191 044**
**US - A - 4 340 695**

(73) Patentinhaber: **CIBA-GEIGY AG, Klybeckstrasse 141, CH-4002 Basel (CH)**

(72) Erfinder: **Steinberg, David H., 2216 Wilson Avenue, New York New York 10469 (US)**
Erfinder: **Luzzi, John J., Ann Road, Carmel New York 10512 (US)**
Erfinder: **Cortolano, Frank P., 3 East Maple Street, Valhalla New York 10595 (US)**

(74) Vertreter: **Zumstein, Fritz jun., Dr. et al, Dr. F. Zumstein sen. Dr. E. Assmann Dr. R. Koenigsberger Dipl.-Ing. F. Klingseisen Dr. F. Zumstein jun. Bräuhausstrasse 4, D-8000 München 2 (DE)**

EP 0 140 298 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft neue Hydroxybenzylthioderivate, deren Verwendung als Stabilisatoren sowie das damit stabilisierte organische Material.

Organische polymere Verbindungen wie Kunststoffe oder Harze, sowie Schmierstoffe oder Mineralöle unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophoren führen. Weiterhin müssen Probleme der Verarbeitungsstabilität und der Substratverträglichkeit beachtet werden.

Überraschenderweise ist jetzt gefunden worden, dass 4-Hydroxybenzylthioaldehyde und 4-Hydroxybenzylthioketone eine ungewöhnliche Kombination wünschenswerter Eigenschaften besitzen, wodurch diese Stoffe zu besonders effektiven und nützlichen Stabilisatoren werden. Die erfindungsgemässen Verbindungen erweisen sich als besonders effektiv in Polyolefinen, schlagfestem Polystyrol, Elastomeren wie Polybutadien oder Styrol-Butadien-Elastomeren, bei denen der Erhalt der Elastizität, die Verhinderung von Vernetzungsreaktionen, von Verfärbung, von Geruchsbildung und von Ausschwitzen des Stabilisators grundlegende Qualitätsanforderungen darstellen.

Die Erfindung betrifft Verbindungen der Formel I

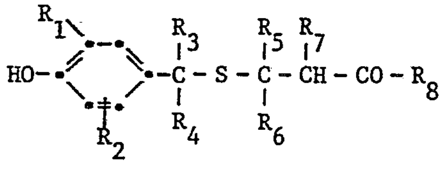 (I),

worin $R_1$, $R_2$, $R_3$, $R_4$, $R_5$, $R_6$, $R_7$ und $R_8$ unabhängig voneinander Wasserstoff, $C_1$-$C_{12}$Alkyl, $C_5$-$C_6$Cycloalkyl, Phenyl, das gegebenenfalls mit $C_1$-$C_{12}$Alkyl substituiert ist, oder $C_7$-$C_9$Aralkyl, das gegebenenfalls mit $C_1$-$C_{12}$Alkyl substituiert ist, bedeuten.

Bevorzugt werden Verbindungen der Formel I, worin $R_2$ sich in ortho-Position zur phenolischen Hydroxygruppe befindet.

Die Gruppen $R_1$ bis $R_8$ bedeuten als $C_1$-$C_{12}$-Alkyl geradkettiges oder verzweigtes Alkyl wie beispielsweise Methyl, Ethyl, n-Propyl, Isopropyl, n-Butyl, sek.-Butyl, tert.-Pentyl, n-Hexyl, 2-Ethylhexyl, n-Octyl, n-Decyl, n-Dodecyl oder 1,1,3,3-Tetramethylbutyl. Besonders bevorzugt werden die Reste $R_1$ bis $R_8$ als $C_1$-$C_8$ Alkyl und insbesondere als Methyl oder tert.-Butyl. Als $C_7$-$C_9$Aralkyl bedeuten die Reste $R_1$ bis $R_8$ beispielsweise Benzyl, α-Methylbenzyl oder α,α-Dimethylbenzyl. Als substituiertes Phenyl sind sie beispielsweise Tolyl, Mesityl oder Xylyl.

Bevorzugt werden Verbindungen der Formel I, worin $R_1$ tert.-Butyl, $R_2$ Methyl oder tert.-Butyl, $R_3$ Wasserstoff oder Methyl, $R_4$ Wasserstoff, Methyl oder Isopropyl und $R_5$ bis $R_8$ unabhängig voneinander Wasserstoff oder Methyl bedeuten.

Die erfindungsgemässen Stabilisatoren werden hergestellt, indem man ein Merkaptan der Formel II mit einer ungesättigten Carbonylverbindung der Formel III in Gegenwart eines Katalysators umsetzt.

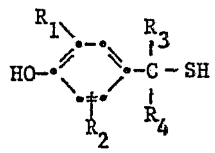 (II) 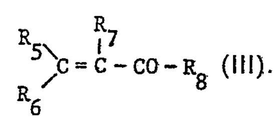 (III).

Die Reste $R_1$ bis $R_8$ haben die gleiche Bedeutung wie oben definiert.

Als Katalysator lassen sich Trialkylamine, wie beispielsweise Triethylamin, oder Pyridin, Natriumhydroxid, Kaliumhydroxid oder Natriumbicarbonat verwenden. Die Synthese kann in unterschiedlichen Lösungsmitteln durchgeführt werden. Es können Alkoholen, Kohlenwasserstoffe, Ether, chlorierte Kohlenwasserstoffe, Aromaten und weitere Lösungsmittel verwendet werden. Es kann aber auch lösungsmittelfrei gearbeitet werden. Die Reaktionen werden im Temperaturbereich zwischen −10° C und 150° C durchgeführt. Bevorzugt wird der Bereich zwischen 20° C und 125° C. Die Ausgangsmaterialien sind im Handel oder können mit Hilfe von Standardmethoden erhalten werden.

Die erfindungsgemässen Verbindungen eignen sich besonders zum Stabilisieren von organischen Materialien wie beispielsweise Kunststoffen, Polymeren, Harzen, Mineralölen oder synthetischen Ölen.

Die Erfindung betrifft daher auch Zusammensetzungen enthaltend ein gegen thermischen, oxidativen oder strahlungsinduzierten Abbau empfindliches organisches Material und mindestens eine Verbindung der Formel I.

Bevorzugt werden Zusammensetzungen, welche als organisches Material ein Polymeres oder ein Schmierstoff auf Mineralölbasis oder einen synthetischen Schmierstoff enthalten.

Enthält die erfindungsmässe Zusammensetzung ein Polymeres als organisches Material, so sind insbesondere Polyolefine wie beispielsweise Polyethylen oder Polypropylen, sowie Polystyrol, besonders bevorzugt schlagfestes Polystyrol, Acrylnitril-Butadien-Styrol-Copolymer (ABS) oder

Elastomere wie beispielsweise Ethylen-Propylen-Copolymer (EPM), Ethylen-Propylen-Dien-Kautschuk (EPDM), Styrol-Butadien-Kautschuk (SBR) oder Nitrilkautschuk gemeint.

Ebenfalls bevorzugt werden Zusammensetzungen, die als organisches Material ein Mineralöl oder einen synthetischen Schmierstoff enthalten.

Die in Frage kommenden Schmierstoffe sind dem Fachmann geläufig und z.B. im «Schmiermittel Taschenbuch (Hüthig Verlag, Heidelberg, 1974)» beschrieben.

Ein weiterer Gegenstand der Erfindung ist die Verwendung von Verbindungen der Formel I als Stabilisatoren für organisches Material gegen dessen Schädigung durch Einwirkung von Sauerstoff, Wärme, Licht oder energiereiche Strahlung.

Generell kann man die Stabilisatoren für folgende polymere Substrate verwenden:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B. Styrol-Butadien, Styrol-Acrylnitril, Styrol-Alkyl-Methacrylat, Styrol-Butadien-Ethylacrylat, Styrol-Maleinhydrid, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Ethyl-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie z.B. Acrylnitril-Butadien-Styrol (ABS), Methylmethacrylat-Butadien-Styrol (MBS), Acrylnitril-Styrol-Acrylester (ASA) oder Pfropfcopolymere von Acrylnitril und Styrol auf Ethylen-Propylen-Dien-Kautschuk.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z.B. Ethylenoxid enthalten.

13. Polyphenylenoxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z.B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-[2,2-(4-hydroxyphenyl)-propan]-terephthalat, Polyhydroxybenzoate, sowie Block-Polyether-ester, die sich von Polyethern mit Hydroxylendgruppen ableiten.

18. Polycarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z.B. von Bis-glycidylethern, von cycloaliphatischen Diepoxiden oder von aromatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z.B. Polypropylen/Ethylen-Propylen-Dien-Kautschuk (PP/EPDM), Polycaprolactam/Ethylen-Propylen-Dien-Kautschuk (Polyamid 6/EPDM), oder Acrylnitril-Butadien-Styrol, Polyvinylchlorid/Ethylenvinylacetat-Copolymer, Polyvinylchlorid/Acrylnitril-Butadien-Styrol, Polyvinylchlorid/Methylmethacrylat-Butadien-Styrol, Polycarbonat/Acrylnitril-Butadien-Styrol, Polybutylenterephthalat/Acrylnitril-Butadien-Styrol.

28. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie z.B. als Weichmacher für Polymere oder als Spinnpräparationen Anwendung finden, sowie deren wässerige Emulsionen.

29. Wässerige Emulsionen natürlicher oder synthetischer Kautschuke, wie z.B. Naturkautschuk-Latex oder Latices von carboxylierten Styrol-Butadien-Copolymeren.

Die Erfindung betrifft weiterhin ein Verfahren zum Stabilisieren von organischem Material gegen oxidativen, thermischen oder strahlungsinduzierten Abbau, dadurch gekennzeichnet, dass man dem Material mindestens eine Verbindung der Formel I wie oben beschrieben zusetzt.

Die erfindungsgemässen Stabilisatoren werden dem jeweiligen Substrat in Mengen von 0,01 bis 5 Gew.% bezogen auf das stabilisierte Material zugemischt. Je nach Verwendung und Substrat wird die Stabilisatormenge variiert. Bevorzugt verwendet man 0,05 bis 2 Gew.% des Stabilisators, besonders bevorzugt 0,1 bis 1 Gew.%.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit einem pulverförmigen Polymeren gemischt werden, oder eine Emulsion oder eine Suspension des Stabilisators kann mit einer Lösung, Suspension oder Emulsion des Polymeren vermischt werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

In der Praxis können die Verbindungen der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z.B. weitere aminische Antioxidantien, Metallpassivatoren, Rostinhibitoren, Viskositätsindex-Verbesserer, Stockpunkterniedriger, Dispergiermittel/Tenside und Verschleissschutzadditive.

Als weitere Additive, mit denen zusammen die erfindungsgemäss verwendeten Stabilisatoren eingesetzt werden können, sind beispielsweise zu nennen:

*1. Antioxidantien*

*1.1. Alkylierte Monophenole*

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol.

*1.2. Alkylierte Hydrochinone*

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol
2,5-Di-tert.butyl-hydrochinon
3,5-Di-tert.butyl-4-hydroxyamid
Tris-(3,5-di-tert.butyl-4-hydroxyphenyl)-phosphit
3,5-Di-tert.butyl-4-hydroxyphenylstearat
Bis-(3,5-di-tert.butyl-4-hydroxyphenyl)-adipat.

*1.3. Hydroxylierte Thiodiphenylether*

2,2'-Thio-bis-(6-tert.butyl-4-methylphenol)

2,2'-Thio-bis-(4-octylphenol)
4,4'-Thio-bis-(6-tert.butyl-3-methylphenol)
4,4'-Thio-bis-(6-tert.butyl-2-methylphenol)
4,4'-Thio-bis-(3,6-di-sek.amylphenol)
4,4'-Bis-(2,6-dimethyl-4-hydroxyphenyl)-disulfid.

*1.4. Alkyliden-Bisphenole*

2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercapto-butan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methylbenzyl)-6-tert.butyl-4-methyl-phenyl]-terephthalat
1,1-Bis-(3,5-dimethyl-2-hydroxyphenyl)-butan
2,2-Bis-(3,5-di-tert.butyl-4-hydroxyphenyl)-propan
1,1,5,5-Tetra-(5-tert.butyl-4-hydroxy-2-methylphenyl)-pentan.

*1.5 Benzylverbindungen*

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz
1,4-Bis-(3,5-di-tert.butyl-4-hydroxybenzyl)-2,3,5,6-tetramethylbenzol
2,4,6-Tris-(3,5-di-tert.butyl-4-hydroxybenzyl)-phenol.

*1.6 Acylaminophenole*

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

*1.7 Ester der β-(3,5-Di-tert.butyl-4-hydroxyphenyl)-propionsäure* mit ein- oder mehrwertigen Alkoholen, wie z.B. mit

| | |
|---|---|
| Methanol | Triethylenglycol |
| Octadecanol | Pentaerythrit |
| 1,6-Hexandiol | Tris-hydroxyethyl-isocyanurat |
| Neopentyglycol | |
| Thiodiethylenglycol | Di-hydroxyethyl-oxalsäurediamid |
| Ethanol | |
| Ethylenglycol | 1,9-Nonandiol |
| Trimethylhexandiol | 1,2-Propandiol |
| Trimethylolpropan | 3-Thia-pentadecanol |
| Diethylenglycol | Trimethylolethan |

4-Hydroxymethyl-1-phospha-2,6,7-trioxabicyclo-[2,2,2]-octan.

*1.8 Ester der β-(5-tert.butyl-4-hydroxy-3-methylphenyl)-propionsäure* mit ein- oder mehrwertigen Alkoholen, wie z.B. mit

| | |
|---|---|
| Methanol | Triethylenglycol |
| Octadecanol | Pentaerythrit |
| 1,6-Hexandiol | Tris-hydroxyethyl-isocyanurat |
| Neopentyglycol | |
| Thiodiethylenglycol | Di-hydroxyethyl-oxalsäurediamid |
| Ethanol | |
| Ethylenglycol | 1,9-Nonandiol |
| 3-Thia-undecanol | 1,2-Propandiol |
| Trimethylolpropan | 3-Thia-pentadecanol |
| Diethylenglycol | Trismethylolethan |

4-Hydroxy-1-phospha-2,6,7-trioxabicyclo-[2,2,2]-octan.

*1.9 Amide der β-(3,5-Di-tert.butyl-4-hydroxyphenyl)-propionsäure*, wie z.B.

N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin
N,N'-Di-(3,5-di-tert.butyl-4-hydroxyphenylpropionyl)-hexamethylendiamin
N,N'-Bis-(3,5-di-tert.butyl-4-hydroxyphenylpropionyl)-hydrazin.

*2. UV-Absorber und Lichtschutzmittel*

*2.1. 2-(2'-Hydroxyphenyl)-benztriazole*, wie z.B. das 5'-Methyl-, 3',5'-Di-tert.butyl-, 5'-Tert.butyl-,5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-octoxy-, 3',5'-Di-tert.-amyl-, 3',5'-Bis-(α,α-dimethylben-

zyl)-, 3'-Methylbenzyl-5'-methyl-5-chlor-, 4'-Hydroxy-, 4'-Methoxy-, 3'-Methyl-5'-carbomethoxyethyl-, 3',5'-Bis-(α,α-dimethylbenzyl)-5-chlor-, 3',5'-Di-tert.octylphenyl, 3',5'-Di-tert.-octylphenyl-5-chlor- oder 5-Chlor-3',5'-di-tert. amyl-Derivate.

*2.2 2-Hydroxybenzophenone*, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

*2.3 Ester von gegebenenfalls substituierten Benzoesäuren*, wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester oder der n-Octadecylester oder der 2-Methyl-4,5-di-tert.butylester.

*2.4 Acrylate*, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

*2.5 Nickelverbindungen*, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

*2.6 Sterisch gehinderte Amine*, wie z.B. Bis-(2,2,6,6-tetramethylpiperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester, Kondensationsprodukt aus 1-Hydroxy-ethyl-2,2,6,6-Tetramethyl-4-hydroxypiperidin und Bernsteinsäure, Kondensationsprodukt aus N,N'-(2,2,6,6-Tetramethylpiperidyl)-hexamethylendiamin und 4-tert.octylamino-2,6-dichlor-1,3,5-s-triazin,
Tris-(2,2,6,6-tetramethylpiperidyl)-nitrilotriacetat,
Tetrakis-(2,2,6,6-tetramethyl-4-piperidyl)-1,2,3,4-butantetracarbonsäure,
1,1'-(1,2-ethandiyl)-bis-(3,3,5,5-tetramethyl-piperazinon)
4-Benzoyl-2,2,6,6-tetramethylpiperidin,
4-Stearyl-oxy-2,2,6,6-tetramethylpiperidin,
3-n-Octyl-7,7,9,9-tetramethyl-1,3,8-triaza-spiro[4,5]decan-2,4-dion.

*2.7 Oxalsäurediamide*, wie z.B. 4,4'-Di-octyloxy-oxanilid, 2,2'-Di-octyloxy-5,5'-di-tert.butyloxanilid, 2,2'-Di-dodecyloxy-5,5'-di-tert.butyloxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

*3. Metalldesaktivatoren*, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bisbenzyliden-oxalsäuredihydrazid.

*4. Phosphite und Phosphonite*, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythrit-diphosphit, Tristearyl-sorbit-triphosphit, Tetrakis-(2,4-di-tert.butylphenyl)-4,4'-biphenylendiphosphonit, 3,9-Isodecyloxy-2,4,8,10-tetraza-3,9-diphospha-spiro-[5,5]-undecan und Tri-(4-hydroxy-3,5-di-tert.butylphenyl)-phosphit.

*5. Peroxidzerstörende Verbindungen*, wie z.B. Ester der β-Thio-dipropionsäure, beispielsweise der Lauryl-, Stearyl-, Myristyl- oder Tridecylester, Mercaptobenzimidazol, das Zinksalz des 2-Mercapto-benzimidazols, Zink-dibutyl-dithiocarbamat, Dioctadecyldisulfid, Pentaerythrit-tetrakis-(β-dodecylmercapto)-propionat.

*6. Polyamidstabilisatoren*, wie z.B. Kupfersalze in Kombination mit Iodiden und/oder Phosphorverbindungen und Salze des zweiwertigen Mangans.

*7. Basische Co-Stabilisatoren*, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinkbrenzcatechinat.

*8. Nukleierungsmittel*, wie z.B. 4-tert.Butylbenzoesäure, Adipinsäure, Diphenylessigsäure.

*9. Füllstoffe und Verstärkungsmittel*, wie z.B. Calciumcarbonat, Silikate, Glasfasern, Asbest, Talk, Kaolin, Glimmer, Bariumsulfat, Metalloxide und -hydroxide, Russ, Graphit.

*10. Sonstige Zusätze*, wie z.B. Weichmacher, Gleitmittel, Emulgatoren, Pigmente, Optische Aufheller, Flammschutzmittel, Antistatika, Treibmittel.

*11. Aminoaryl Derivate* wie beispielsweise: Phenyl-1-naphthylamin, Phenyl-2-naphthylamin, N,N'-Diphenyl-p-phenylendiamin, N,N'-Di-2-naphthyl-p-phenylendiamin, N,N'-Dinaphthyl-p-phenylendiamin, N,N'-Di-sek.butyl-p-phenylendiamin, 6-Ethoxy-2,2,4-trimethyl-1,2-dihydrochinolin, 6-Dodecyl-2,2,4-trimethyl-1,2-dihydrochinolin, Mono- und Dooctyliminodibenzyl, polymerisiertes 2,2,4-Trimethyl-1,2-dihydrochinolin, octyliertes Diphenylamin, nonyliertes Diphenylamin, N-Phenyl-N'-cyclohexyl-p-phenylendiamin, N-Phenyl-N'-isopropyl-p-phenylendiamin, N,N'-Di-sek.octyl-p-phenylendiamin, N-Phenyl-N'-sek.octyl-p-phenylendiamin, N,N'-Di-(1,4-dimethylpentyl)-p-phenylendiamin, N,N'-Di-(1,4-dimethylpentyl)-p-phenylendiamin, N,N'-Dimethyl-N,N'-di(sek.octyl)-p-phenylendiamin, 2,6-Dimethyl-4-methoxyanilin, 4-Ethoxy-N-sek.butylanilin, Kondensationsprodukt von Diphenylamin und Aceton, Aldol-1-naphthylamin und Phenothiazin.

*12. Metallpassivatoren:*

für Kupfer, z.B.:
Benztriazol, Tetrahydrobenztriazol, 2-Mercaptobenzthiazol, 2,5-Dimercaptothiadiazol, Salicyliden-propylendiamin, Salze von Salicylaminoguanidin.

*13. Rost-Inhibitoren:*

a) Organische Säuren, ihre Ester, Metallsalze und Anhydride, z.B.:
N-Oleoyl-sarcosin, Sorbitan-mono-oleat, Bleinaphthenat, Dodecenylbernsteinsäure-anhydrid, Alkenylbernsteinsäure-Halbester, 4-Nonyl-phenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen z.B.:
Substituierte Imidazoline und Oxazoline.
c) Phosphorhaltige Verbindungen, z.B.:
Aminsalze von Phosphorsäurepartialestern.
d) Schwefelhaltige Verbindungen, z.B.:
Barium-dinonylnaphthalin-sulfonate, Calciumpetroleum-sulfonate.

*14. Viskositätsindex-Verbesserer:*

Polymethacrylate, Vinylpyrrolidon/Methacrylat-Copolymere, Polybutene, Olefin-Copolymere, Styrol/Acrylat-Copolymere.

*15. Stockpunkterniedriger:*

Polymethacrylat, alkylierte Naphthalinderivate.

*16. Dispergiermittel/Tenside:*

Polybutenylbernsteinsäure-imide, Polybutenylphosphonsäurederivate, basische Magnesium-, Calcium-, und Bariumsulfonate und -phenolate.

*17. Verschleissschutz-Additive:*

Schwefel und/oder Phosphor und/oder Halogenenthaltende Verbindungen, wie geschwefelte pflanzliche Öle, Zinkdialkyldithiophosphate, Tritolyl-phosphat, chlorierte Paraffine, Alkyl- und Aryldisulfide.

*Beispiele:*

*Beispiel 1:* Herstellung von 3-(3,5-Di-tert.butyl-4-hydroxybenzylthio)-butyraldehyd.

Zu einer Lösung aus 25,2 g (0,1 Mol) 3,5-Di-tert.butyl-4-hydroxybenzylmercaptan und 7,0 g (0,1 Mol) Crotonaldehyd in 20 ml Methanol gibt man unter Rühren 0,5 ml Triethylamin. Die Reaktion ist exotherm und die Temperatur steigt von Raumtemperatur auf etwa 40° C an. Nach Beendigung der Umsetzung wird weitere drei Stunden auf 50° C gehalten und anschliessend auf Raumtemperatur abkühlen gelassen. Methanol und Triethylamin werden im Vakuum abgezogen und der Rückstand aus Hexan umkristallisiert. Man erhält 25,0 g eines weissen Feststoffs vom F.P. 55°-60° C.

*Beispiel 2:* Herstellung von 3-(3,5-Di-tert.butyl-4-hydroxybenzylthio)-3-phenylpropionaldehyd.

Zu einer Lösung aus 12,6 g (0,05 Mol) 3,5-Di-tert.butyl-4-hydroxybenzylmercaptan und 6,6 g (0,05 Mol) Zimtaldehyd in 25 ml Hexan gibt man unter Rühren 0,5 ml Triethylamin. Die Reaktionslösung wird anschliessend für sechs Stunden auf 50° C erhitzt und dann weitere 15 Stunden bei Raumtemperatur stehengelassen. Hexan und Triethylamin werden im Vakuum abgezogen und der feste Rückstand chromatographisch gereinigt. Man erhält 3,9 g eines schwach gelben Feststoffs vom F.P. 75°-79° C.

*Beispiel 3:* Herstellung von 3-(3,5-Di-tert.butyl-4-hydroxybenzylthio)-propionaldehyd.

Zu einer Lösung aus 12,6 g (0,05 Mol) 3,5-Di-tert.butyl-4-hydroxybenzylmercaptan und 2,8 g (0,05 Mol) Acrolein in 20 ml Hexan werden unter Rühren 0,55 ml Triethylamin zugefügt. Nach Anwärmen der Reaktionslösung beginnt die exotherme Umsetzung und die Temperatur steigt auf 55° C. Nach 10 bis 15 Minuten fällt die Temperatur auf 35° C. Danach erhitzt man die Lösung für drei Stunden auf etwa 60° C, lässt sie anschliessend abkühlen und über Nacht stehen. Hexan und Triethylamin werden im Vakuum abgezogen und der Rückstand chromatographisch gereinigt. Man erhält 2,9 g eines hellgelben syrupartigen Produktes.

| *Analyse:* | | | |
|---|---|---|---|
| Berechnet: | C 70,08 | H 9,15 | S 10,4% |
| Gefunden: | C 70,4 | H 8,9 | S 10,3% |

*Beispiel 4:* Herstellung von 3-(3-Methyl-5-tert.-butyl-4-hydroxybenzylthio)-butyraldehyd.

Zu einer Lösung aus 21,03 g (0,1 Mol) 3-Methyl-5-tert.butyl-4-hydroxybenzylmercaptan in 25 ml Tetrahydrofuran tropft man unter Rühren eine Lösung aus 0,2 g (0,002 Mol) Triethylamin in 10,5 g (0,15 Mol) Crotonaldehyd. Die Zugabe erfolgt innerhalb von 15 Minuten bei Raumtemperatur. Es erfolgt eine leicht exotherme Reaktion und die Temperatur steigt auf 38° C. Nach Beendigung der Erwärmung erhitzt man weitere vier Stunden auf 50° C und kontrolliert den Endpunkt der Reaktion mit Hilfe der Dünnschichtchromatographie.

Nach Abkühlen auf Raumtemperatur lässt man die Reaktionslösung über Nacht stehen, nimmt anschliessend mit 150 ml Ether auf und wäscht sukzessive mit Wasser und gesättigter NaCl-Lösung. Die etherische Phase wird über einem Molekularsieb getrocknet, abfiltriert und anschliessend im Rotationsverdampfer eingeengt. Der verbleibende Rückstand ist hellgelb und syrupähnlich. Er wiegt 23,9 g und besteht im wesentlichen aus reinem Produkt (dünnschichtchromatographisch kontrolliert).

*Analyse:*

| | | | |
|---|---|---|---|
| Berechnet: | C 68,53 | H 8,63 | S 11,43% |
| Gefunden: | C 68,8 | H 8,7 | S 11,4 % |

*Beispiel 5:* Herstellung von 4-(3,5-Di-tert.butyl-4-hydroxybenzylthio)-5-methylhexan-2-on.

Zu einer Lösung aus 13,0 g (0,051 Mol) 3,5-Di-tert.butyl-4-hydroxybenzylmercaptan und 7,2 g (0,051 Mol) 5-Methylhex-3-en-2-on in 15 ml Hexan gibt man unter Rühren 0,5 g Triethylamin. Die Zugabe erfolgt bei Raumtemperatur und die Reaktionslösung erwärmt sich auf 31° C. Nach Beendigung der exothermen Reaktion (ca. 15 Minuten) wird die Reaktionslösung auf 40° C bis 45° C erwärmt und für zwei Tage auf dieser Temperatur gehalten. Nach Abkühlen wird das Lösungsmittel im Vakuum abgezogen. Der Rückstand wird chromatographisch gereinigt und man erhält ein weisses syrupartiges Produkt.

*Analyse:*

| | | | |
|---|---|---|---|
| Berechnet: | C 72,47 | H 9,95 | S 8,8% |
| Gefunden: | C 72,8 | H 10,0 | S 8,7% |

*Beispiel 6:* Herstellung von 4-Methyl-4-(3,5-di-tert.butyl-4-hydroxybenzylthio)-pentan-2-on.

Zu einer Lösung aus 13,0 g (0,051 Mol) 3,5-Di-tert.butyl-4-hydroxybenzylmercaptan und 5,0 g (0,051 Mol) Mesityloxid in 20 ml Hexan werden unter Rühren 0,5 g Triethylamin zugetropft. Die Zugabe erfolgt bei Raumtemperatur. Anschliessend wird das Reaktionsgemisch für sechs Stunden auf 50° C erwärmt und danach für eine weitere halbe Stunde auf 75° C. Nach dem Abkühlen wird über Nacht stehengelassen. Am nächsten Tag fügt man einige Tropfen Piperidin zu der Reaktionslösung und erhitzt für weitere vier Tage auf 75° C bis 85° C. Nach Beendigung dieser Behandlung wird abkühlen gelassen und die flüchtigen Bestandteile im Vakuum abgezogen. Der Rückstand wird chromatographisch gereinigt und man erhält ein gelbstichiges Produkt vom F.P. 60° C bis 68° C.

*Beispiel 7:* Herstellung von 4-(3-Methyl-5-tert.-butyl-4-hydroxybenzylthio)-5-methylhexan-2-on.

Zu einer Lösung aus 10,7 g (0,051 Mol) 3-Methyl-5-tert.butyl-4-hydroxy-benzyl-mercaptan und 7,2 g (0,051 Mol) 5-Methyl-hex-3-en-2-on in 25 ml Hexan tropft man bei Raumtemperatur und unter Rühren 0,5 g Triethylamin. Die Reaktionslösung wird dann siebeneinhalb Stunden auf 50° C bis 55° C erwärmt und anschliessend bei Raumtemperatur über Nacht stehengelassen. Am nächsten Tag werden alle flüchtigen Bestandteile im Vakuum abgezogen. Der Rückstand wird chromatographisch gereinigt und man erhält ein weisses halbfestes Produkt vom F.P. 30° C bis 32° C.

*Beispiel 8:* In diesem Beispiel wird die stabilisierende Wirkung der erfindungsgemässen Verbindungen in schlagfestem Polystyrol demonstriert.

a) Herstellung der Proben: Man stellt eine Lösung von 8 Gew.% Polybutadien-Kautschuk (Firestone DIENE 55) in monomerem Styrol her, indem man beide Komponenten in einer Quetschmühle mischt. Gleichzeitig wird in diesem Arbeitsgang der jeweils gewünschte Anteil des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, das mit einem Wendelrührer ausgerüstet ist. Da die meisten IPS Blockpolymerisationen thermisch initiiert werden, wird in der vorliegenden Laboratoriumsversion kein Starter zugesetzt. Die Reaktion wird unter Stickstoff und unter Erwärmen durchgeführt. Der Reaktor wird innerhalb einer halben Stunde auf 121° C erwärmt, und diese Temperatur wird aufrechterhalten, bis zwischen 30 und 35% des Monomeren umgesetzt sind (Zeitdauer ca. 2,5 Stunden). Während der Polymerisation wird heftig gerührt, und die Rührgeschwindigkeit wird so eingestellt, dass Polymerpartikel von 2 bis 4 µm Grösse entstehen. Anschliessend werden die Reaktionsgefässe dem Reaktor entnommen, im Stickstoffstrom geöffnet und in ein Wirbelschichtsandbad gestellt, um die Polymerisation zu vervollständigen. Die Gefässe werden im Sandbad folgendermassen erhitzt:

eine Stunde bei 100° C, um die Anfangstemperatur vorzugeben, eine weitere Stunde, um 140° C zu erreichen, und anschliessend weitere 8 Stunden, wobei die Temperatur jede Stunde um 10° C erhöht wird, bis schliesslich ein Maximum von 220° C erreicht ist. Nach dem Abkühlen werden die Polymerisationsgefässe zerbrochen und das Glas enffernt. Ein einziger Polymerblock wiegt im Mittel etwas über 600 g. Der Block wird in einem Vakuumofen bei 200° C für 45 Minuten bei einem Druck von 1,33 mbar belassen, um flüchtige Bestandteile zu entfernen. Nach dieser Behandlung wird der Block sofort in einer heizbaren hydraulischen Presse bei 205° C zwischen zwei Aluminiumfolien zu einem dicken Stab gepresst (drei Minuten Erhitzen, fünf Minuten ungeheizt). Der Stab wird mittels einer Bandsäge zerkleinert und anschliessend granuliert. Alle Polymermischungen werden bei 205° C extrudiert und dann zerkleinert. Die Pellets werden unter Druck bei 205° C in dehnbare 3,2 mm dicke Streifen verformt.

b) Testverfahren: Diese Probestreifen werden dann bei 150° C in einem Umluftofen gealtert, wobei sie auf Glasplatten, die sich wiederum auf rotierenden Trägern befinden, aufgebracht sind. Eine weitere Charge von Probestreifen wird auf dieselbe Weise bei 80° C im Ofen gealtert. Nach

bestimmten Zeitintervallen wird der Yellowness Index der Proben gemäss ASTM D-1925-63T bestimmt, und es wird die Dehnbarkeit gemäss ASTM D-638 bestimmt (Instron Tensile Testing Apparatus, Instron Engineering Corporation, Massachusetts; Ziehegeschwindigkeit: 55 mm/Minute).

In der folgenden Tabelle Ia sind die Ergebnisse der Dehnbarkeitsmessungen und der Messungen des Yellowness Indexes von bei 80° C ofengealterten Proben angegeben, während Tabelle 1b) Messwerte von bei 150° C ofengealterten Proben enthält.

*Tabelle 1a:* Dehnbarkeitsmessungen und Yellowness Index von bei 80°C ofengealterten Proben mit und ohne Stabilisator

| Additiv | Stabilisatormenge (Gew.%) | Dehnbarkeit der Probe (%) und Dauer der Hitzebelastung (h) | | | | |
|---|---|---|---|---|---|---|
| | | 0h | 300h | 600h | 900h | 1200h |
| —*) | — | — | — | — | — | 4 |
| Produkt von Beispiel 4 | 0,1 | 33 | 20 | 11,6 | 9,3 | 11,2 |
| Produkt von Beispiel 5 | 0,1 | 58 | 35 | 24,2 | 19,8 | 13,7 |
| Produkt von Beispiel 6 | 0,1 | 35 | 21 | 14,1 | 11,1 | 9,4 |
| Produkt von Beispiel 7 | 0,1 | 44 | 30 | 22 | 20,5 | 16,7 |

| Additiv | Stabilisatormenge (Gew.%) | Yellowness Index und Dauer der Hitzebelastung (h) | | | | |
|---|---|---|---|---|---|---|
| | | 0h | 300h | 600h | 900h | 1200h |
| —*) | — | −12 | — | — | — | 47 |
| Produkt von Beispiel 4 | 0,1 | −9 | 4 | 11 | 18 | 20 |
| Produkt von Beispiel 5 | 0,1 | −10 | −2 | 10 | 20 | 28 |
| Produkt von Beispiel 6 | 0,1 | −5 | −8 | 10 | 16 | 19 |
| Produkt von Beispiel 7 | 0,1 | −7 | −0,6 | 6 | 10 | 12 |

*Tabelle 1b:* Dehnbarkeitsmessungen und Yellowness Index von bei 150°C ofengealterten Proben mit und ohne Stabilisator

| Additiv | Stabilisatormenge (Gew.%) | Dehnbarkeit der Probe (%) und Dauer der Hitzebelastung (h) | | | | |
|---|---|---|---|---|---|---|
| | | 0h | 0,5h | 1,0h | 1,5h | 2,0h |
| —*) | — | — | — | 12 | — | 6 |
| Produkt von Beispiel 1 | 0,1 | 32 | 30 | 32 | 17 | 14 |
| Produkt von Beispiel 4 | 0,1 | 33 | 29 | 24 | 19 | 11 |
| Produkt von Beispiel 5 | 0,1 | 58 | 48 | 35 | 30 | 21 |
| Produkt von Beispiel 6 | 0,1 | 35 | 31 | 25 | 19 | 14 |
| Produkt von Beispiel 7 | 0,1 | 44 | 33 | 29 | 24 | 22 |

| Additiv | Stabilisatormenge (Gew.%) | Yellowness Index und Dauer der Hitzebelastung (h) | | | | |
|---|---|---|---|---|---|---|
| | | 0h | 0,5h | 1,0h | 1,5h | 2,0h |
| —*) | — | −12 | — | −7 | — | 1 |
| Produkt von Beispiel 1 | 0,1 | −13 | −9 | −10 | −4 | −3 |
| Produkt von Beispiel 4 | 0,1 | −9 | −6 | −7 | −4 | −2 |
| Produkt von Beispiel 5 | 0,1 | −10 | −9 | −7 | −7 | −2 |
| Produkt von Beispiel 6 | 0,1 | −5 | −3 | −1 | 3 | 5 |
| Produkt von Beispiel 7 | 0,1 | −7 | −5 | −3 | −2 | −1 |

*) Probe enthält 0,025 Gew.% Octadecyl-3-(3,5-di-tert. butyl-4-hydroxyphenyl)-propionat.

## Patentansprüche

1. Verbindungen der Formel I

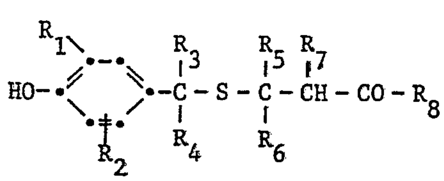

(I),

worin $R_1$, $R_2$, $R_3$, $R_4$, $R_5$, $R_6$, $R_7$ und $R_8$ unabhängig voneinander Wasserstoff, $C_1$-$C_{12}$Alkyl, $C_5$-$C_6$Cycloalkyl, Phenyl, das gegebenenfalls mit $C_1$-$C_{12}$Alkyl substituiert ist, oder $C_7$-$C_9$Aralkyl, das gegebenenfalls mit $C_1$-$C_{12}$Alkyl substituiert ist, bedeuten.

2. Verbindungen der Formel I gemäss Anspruch 1, worin $R_2$ sich in ortho-Position zur phenolischen Hydroxygruppe befindet.

3. Verbindungen der Formel I gemäss Anspruch 2, worin $R_1$ und $R_2$ $C_1$-$C_8$Alkyl bedeuten.

4. Verbindungen der Formel I gemäss Anspruch 2, worin $R_1$ und $R_2$ tert.Butyl bedeuten.

5. Verbindungen der Formel I gemäss Anspruch 2, worin $R_1$ tert.Butyl, $R_2$ Methyl oder tert.Butyl,

$R_3$ Wasserstoff oder Methyl, $R_4$ Wasserstoff, Methyl oder Isopropyl und $R_5$, $R_6$, $R_7$ und $R_8$ unabhängig voneinander Wasserstoff oder Methyl bedeuten.

6. Die Verbindungen 3-(3,5-Di-tert.butyl-4-hydroxybenzylthio)-butyraldehyd, 3-(3,5-Di-tert.-butyl-4-hydroxybenzylthio)-3-phenylpropionaldehyd, 3-(3,5-Di-tert.butyl-4-hydroxybenzylthio)-propionaldehyd, 3-(3-Methyl-5-tert.butyl-4-hydroxybenzylthio)-butyraldehyd, 4-(3,5-Di-tert.-butyl-4-hydroxybenzylthio)-5-methylhexan-2-on, 4-Methyl-4-(3,5-di-tert.butyl-4-hydroxybenzylthio)-pentan-2-on oder 4-(3-Methyl-5-tert.butyl-4-hydroxybenzylthio)-5-methylhexan-2-on gemäss Anspruch 1.

7. Zusammensetzung enthaltend ein gegen oxidativen, thermischen oder strahlungsinduzierten Abbau empfindliches organisches Material und mindestens einen Stabilisator der Formel I gemäss Anspruch 1.

8. Zusammensetzung gemäss Anspruch 7, worin das organische Material ein synthetisches Polymer ist.

9. Zusammensetzung gemäss Anspruch 8, worin das synthetische Polymer ein Polyolefin, schlagfestes Polystyrol, Acrylnitril-Butadien-Styrol, Butadien-Kautschuk, Ethylen-Propylen-Copolymer, Ethylen, Propylen-Dien-Kautschuk, Styrol-Butadien-Kautschuk oder Nitrilkautschuk ist.

10. Verfahren zum Stabilisieren von organischem Material gegen oxidativen, thermischen oder strahlungsinduzierten Abbau, dadurch gekennzeichnet, dass man dem Material mindestens eine Verbindung der Formel I gemäss Anspruch 1 zusetzt.

11. Verwendung von Verbindungen der Formel I gemäss Anspruch 1 als Stabilisatoren für organisches Material gegen dessen Schädigung durch Einwirkung von Sauerstoff, Wärme, Licht oder energiereiche Strahlung.

## Claims

1. A compound of the formula I

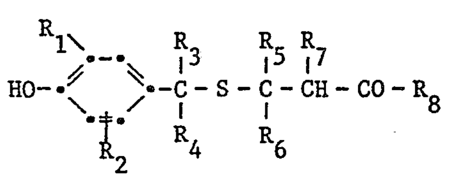

(I)

wherein $R_1$, $R_2$, $R_3$, $R_4$, $R_5$, $R_6$, $R_7$ and $R_8$ are each independently hydrogen, alkyl of 1 to 12 carbon atoms, cycloalkyl of 5 to 6 carbon atoms, phenyl, phenyl substituted by alkyl of 1 to 12 carbon atoms, aralkyl of 7 to 9 carbon atoms or said aralkyl substituted by alkyl of 1 to 12 carbon atoms.

2. A compound of the formula I according to claim 1, wherein $R_2$ is in the ortho-position to the phenolic hydroxyl group.

3. A compound of the formula I according to claim 2, wherein $R_1$ and $R_2$ are alkyl of 1 to 8 carbon atoms.

4. A compound of the formula I according to claim 2, wherein $R_1$ and $R_2$ are tert-butyl.

5. A compound of the formula I according to claim 2, wherein $R_1$ is tert-butyl, $R_2$ is methyl or tert-butyl, $R_3$ is hydrogen or methyl, $R_4$ is hydrogen, methyl or isopropyl and $R_5$, $R_6$, $R_7$ and $R_8$ are each independently hydrogen or methyl.

6. 3-(3,5-di-tert-butyl)-4-hydroxybenzylthio)-butyraldehyde, 3-(3,5-di-tert-butyl-4-hydroxybenzylthio)-3-phenylpropionaldehyde, 3-(3,5-di-tert-butyl-4-hydroxybenzylthio)propionaldehyde, 3-(3-methyl-5-tert-butyl-4-hydroxybenzylthio)butyraldehyde, 4-(3,5-di-tert-butyl-4-hydroxybenzylthio)-5-methylhexan-2-one, 4-methyl-4-(3,5-di-tert-butyl-4-hydroxybenzylthio)pentan-2-one or 4-(3-methyl-5-tert-butyl-4-hydroxybenzylthio)-5-methylhexan-2-one according to claim 1.

7. A composition comprising an organic material subject to oxidative, thermal or radiation-induced degradation and at least one stabiliser of the formula I according to claim 1.

8. A composition according to claim 7, wherein the organic material is a synthetic polymer.

9. A composition according to claim 8, wherein the synthetic polymer is selected from the group consisting of polyolefins, impact-resistant polystyrene, ABS, butadiene rubber, EPM, EPDR, SBR and nitrile rubber.

10. A method of stabilizing an organic material against oxidative, thermal or radiation-induced degradation, which comprises incorporating into said organic material at least one compound of the formula I according to claim 1.

11. Use of a compound of the formula I according to claim 1 for stabilising organic material against the action of oxygen, heat, light or energy-rich radiation.

## Revendications

1. Composés répondant à la formule I:

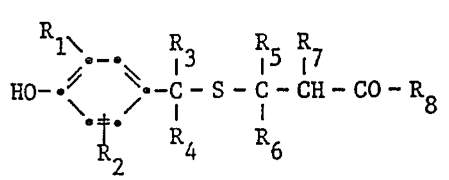

(I)

dans laquelle $R_1$, $R_2$, $R_3$, $R_4$, $R_5$, $R_6$, $R_7$ et $R_8$ représentent chacun, indépendamment les uns des autres, l'hydrogène, un alkyle en $C_1$-$C_{12}$, un cycloalkyle en $C_5$ ou $C_6$, un phényle éventuellement porteur d'un alkyle en $C_1$-$C_{12}$, ou un aralkyle en $C_7$-$C_9$ éventuellement porteur d'un alkyle en $C_1$-$C_{12}$.

2. Composés de formule I selon la revendication 1 dans lesquels $R_2$ se trouve en position ortho relativement au radical hydroxy phénolique.

3. Composés de formule I selon la revendication 2 dans lesquels $R_1$ et $R_2$ représentent chacun un radical alkyle en $C_1$-$C_8$.

4. Composés de formule I selon la revendica-

tion 2 dans lesquels $R_1$ et $R_2$ représentent chacun un radical tert-butyle.

5. Composés de formule I selon la revendication 2 dans lesquels $R_1$ représente un radical tert-butyle, $R_2$ un radical méthyle ou tert-butyle, $R_3$ l'hydrogène ou un radical méthyle, $R_4$ l'hydrogène ou un radical méthyle ou isopropyle et $R_5$, $R_6$, $R_7$ et $R_8$ représentent chacun, indépendamment les uns des autres, l'hydrogène ou un radical méthyle.

6. Composés selon la revendication 1, en l'espèce le (di-tert-butyl-3,5 hydroxy-4 benzylthio)-3 butyraldéhyde, le (di-tert-butyl-3,5 hydroxy-4 benzylthio)-3 phényl-3 propionaldéhyde, le (di-tert-butyl-3,5 hydroxy-4 benzylthio)-3 propionaldéhyde, le (méthyl-3 tert-butyl-5 hydroxy-4 benzylthio)-3 butyraldéhyde, la (di-tert-butyl-3,5 hydroxy-4 benzylthio)-4 méthyl-5 hexanone-2, la méthyl-4 (di-tert-butyl-3,5 hydroxy-4 benzylthio)-4 pentanone-2 et la (méthyl-3 tert-butyl-5 hydroxy-4 benzylthio)-4 méthyl-5 hexanone-2.

7. Composition contenant une matière organique susceptible de se dégrader sous l'action de l'oxydation, de la chaleur ou d'un rayonnement et au moins un stabilisant de formule I selon la revendication.

8. Composition selon la revendication 7 dans laquelle la matière organique est un polymère synthétique.

9. Composition selon la revendication 8 dans laquelle le polymère synthétique est une polyoléfine, du polystyrène haute résilience, de l'acrylonitrile/butadiène/styrène, un caoutchouc de butadiène, un copolymère éthylène/propylène, un caoutchouc éthylène/propylène/diène, un caoutchouc styrène/butadiène ou du caoutchouc nitrile.

10. Procédé pour stabiliser des matières organiques contre la dégradation causée par l'oxydation, la chaleur ou un rayonnement, procédé caractérisé en ce qu'on ajoute à la matière au moins un composé de formule I selon la revendication 1.

11. Application de composés de formule I selon la revendication 1 comme stabilisants pour des matières organiques contre leur dégradation par l'action de l'oxygène, de la chaleur, de la lumière ou d'un rayonnement de haute énergie.